(19) Europäisches Patentamt
European Patent Office
Office européen des brevets

(11) EP 0 557 404 B1

(12) **FASCICULE DE BREVET EUROPEEN**

(45) Date de publication et mention de la délivrance du brevet:
**15.05.1996 Bulletin 1996/20**

(21) Numéro de dépôt: **91920899.1**

(22) Date de dépôt: **12.11.1991**

(51) Int. Cl.[6]: **A61K 31/05**, A61K 31/085, A61K 31/11, A61K 31/22, A61K 31/23, A61K 31/165, A61K 31/19, A61K 31/20, C07C 41/16, C07C 45/51, C07C 45/68

(86) Numéro de dépôt international:
**PCT/FR91/00882**

(87) Numéro de publication internationale:
**WO 92/08450 (29.05.1992 Gazette 1992/12)**

(54) **UTILISATION ANTIVIRALE DE COMPOSE 2,6-DI-t-BUTYLPHENOL SUBSTITUE EN POSITION 4, NOTAMMENT VIS-A-VIS DES VIRUS DE L'HERPES ET DES PAPILLOMAVIRUS**

ANTIVIRALE VERWENDUNG EINER IN POSITION-4 SUBSTITUIERTEN 2,6-DI-T-BUTYLPHENOLVERBINDUNG, BESONDERS GEGEN HERPES- UND PAPILLOMAVIREN

ANTIVIRAL USE OF A 2,6-DI-t-BUTYLPHENOL COMPOUND SUBSTITUTED IN POSITION 4, PARTICULARLY IN RELATION TO HERPESVIRUSES AND PAPILLOMAVIRUSES

(84) Etats contractants désignés:
**BE CH DE ES FR GB IT LI**

(30) Priorité: **12.11.1990 FR 9013984**
**20.02.1991 FR 9102028**

(43) Date de publication de la demande:
**01.09.1993 Bulletin 1993/35**

(73) Titulaire: **FILECO**
**F-75009 Paris (FR)**

(72) Inventeur: **VACHY, Robert**
**F-75009 Paris (FR)**

(74) Mandataire: **Kopacz, William James**
**BREESE-MAJEROWICZ,**
**CNIT-WTC1,**
**B.P. 434**
**F-92053 Paris La Défense (FR)**

(56) Documents cités:

| | |
|---|---|
| **EP-A- 0 061 508** | **EP-A- 0 382 077** |
| **WO-A-91/13626** | **FR-A- 2 507 891** |
| **GB-A- 2 200 113** | **US-A- 4 708 966** |

- **Science, volume 188, no. 4183, 4 avril 1975, W. Snipes et al.: "Butylated hydroxytoluene inactivates lipid-containing viruses", pages 64-65, voir l'article en entier (cité dans la demande)**
- **Symposium on the Pharmacological Effects of Lipids, (The American Oil Chemist's Society, Champaign, Illinois, (US) 1978, W. Snipes et al.: "Hydrophobic alcohols and di-tert-butyl phenols as antiviral agents", pages 63-73, voir l'article en entier (cité dans la demande)**
- **THE NEW ENGLAND JOURNAL OF MEDICINE, vol. 314, no. 10, 6 March 1986; D.M. SHLIAN: "Toxicity of butylated hydroxytoluene", pages 648-649.**
- **FARM. ZH. (Kiev), vol. 30, no. 3, 1975, A.I. TIKHONOV et al.: "Physico-chemical, microbiological and quantitative determination of watersoluble polyphenol propolis preparations", pages 42-48.**
- **WEST. J. MED., vol. 145, no. 2, August 1986, M.W. GROGAN: "Toxicity from BHT ingestion", pages 245-246.**
- **WEST. J. MED., vol. 139, no. 2, August 1983, J.G. LLAURODO. "Beware of phenolic antioxidants (BHT and BHA)", pages 229-230.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 557 404 B1

- SCIENCE, vol. 188, no. 4183, 4 April 1975, W. SNIPES et al.: "Butylated hydroxytoluene inactivates lipid-containing viruses", pages 64-65.
- ANTIVIRAL RESEARCH, vol. 5, no. 5, October 1985; J.T. RICHARDS et al.: "Topical butylated hydroxytoluene treatment of genital herpes simplex virus infections of guinea pigs", pages 281-290.
- DATABASE WPI/L, accession no. 86-313850, Derwent Publications LTD., London, GB
- DATABASE WPI/L, accession no. 83-762896, Derwent Publications LTD., London, GB.
- CHEMICAL ABSTRACTS, vol. 65, 1966, Columbus, Ohio, USA; N.P. KONOVALOVA et al.: "Structure and antileukemic action of a series of substituted phenols", column 9522a.
- JOURNAL OF ORGANIC CHEMISTRY, vol. 21, 1956, G.R. YOHE et al.: "The oxidation of 2,6-di-tert-butyl-4-methylphenol", pages 1289-1292.
- BIOCHEM. J., vol. 78, no. 4, 1961; J.C. DACRE: "The metabolism of 3:5-di-tert-butyl-4-hydroxytoluene and 3:5-di-tert-butyl-4-hydroxybenzoic acid in the rabbit", pages 758-766.
- JOURNAL OF MEDICINAL AND PHARMACEUTICAL CHEMISTRY, vol. 2, no. 2, 1960; R.B. MOFFETT et al.: "Central nervous system depressants - III. 2- and 4-allyloxy-3,5-disubstituted benzoic acids and derivatives", pages 213-227.

## Description

La présente invention a trait à une nouvelle utilisation antivirale de composés de formule I ci-après appartenant à la famille des composés 2,6-di-t-butylphénols substitués en position 4 [autre nomenclature : 3,5-di-t-butyl-4-hydroxybenzènes substitués en position 1] et résultant, d'une manière générale, de l'oxydation du 2,6-di-t-butyl-4-méthylphénol [autre nomenclature : 3,5-di-t-butyl-4-hydroxytoluène, ou 2,6-di-t-butyl-p-crésol.

Cette nouvelle utilisation antivirale se manifeste notamment vis-à-vis des virus à capside lipidique, comme les virus de l'herpès, mais aussi vis-à-vis d'autres virus et notamment des papillomavirus.

On sait que le 2,6-di-t-butyl-4-méthylphénol (en abrégé HG1) possède des propriétés antivirales vis-à-vis des virus de l'herpès, tels que $HSV_1$, qui sont des virus à capside lipidique (en abrégé LCV), notamment du brevet français FR-B-2 507 891, de l'article de W.SNIPES et al., Sciences, 187, pages 64-65, (1975), et de l'article de W.SNIPES et al. intitulé "Hydrophobic Alcohols and Di-tert-butyl Phenols as Antiviral Agents" et publié dans l'ouvrage "Symposium on the Pharmacological Effects of Lipids", pages 63-73, The American Oil Chemists' Society, Champaign, Illinois (1978).

On sait en particulier que les propriétés antivirales du 2,6-di-t-butyl-4-méthylphénol sont si faibles vis-à-vis des virus de l'herpès, qu'il convient de lui associer un adjuvant pour exalter lesdites propriétés (voir FR-B-2 507 891 précité) ou pour obtenir une synergie avec la propolis (voir à cet effet la demande de brevet français No 90 03 093 du 12 mars 1990, de la Demanderesse).

La demande française No 90 03 093 précitée préconise plus particulièrement une association synergétique d'un composant phénol appartenant à l'ensemble des 2,6-di-t-butylphénols [en abrégé BHT, i.e. composés de formule I où R est un groupe hydrocarboné aliphatique en $C_1$-$C_{12}$] avec la propolis suivant un rapport pondéral BHT/propolis de 100/1 à 650/1 et mieux de 135/1 à 560/1 pour le traitement des infections provoquées parles LCV.

On sait par ailleurs que les composés de formule I ci-après, où R est un reste oxygéné, ont déjà été décrits ou sérieusement suggérés en tant que produits d'oxydation de composés BHT (notamment HG1) ou en tant que métabolites de composés BHT (notamment HG1). Voir à cet effet, les articles de G.R. YOHE et al., J. Org. Chem., 21, pages 1289-1292, (1956), T.H. COFFIELD et al., J. Am. Chem. Soc., 79, 5019-5023, (1957), J.C. DACRE, Biochem. J., 78, pages 758-766, (1961), et M. AKAGI et al., Chem. Pharm. Bull. (Tokyo), 10, pages 101-105 et 200-204, (1962).

Il se trouve que, après administration orale voire injectable de HG1, la métabolisation in vivo en composés de formule I, où R est un reste contenant de l'oxygène tel que $CH_2OH$, $OCH_3$ ou COOH, est lente et intervient tardivement. Par suite, les quantités de métabolites formés biologiquement disponibles dans l'organisme, qui ne sont pas éliminées par les voies naturelles, sont à la connaissance de la Demanderesse insuffisantes pour produire les effets antiviraux bénéfiques notamment vis-à-vis des LCV tels que les virus de l'herpès, d'une part, et des papillomavirus, d'autre part.

De plus, lors de l'administration locale de HG1 sur la peau, dans le cas des traitements des infections dermiques provoquées par les virus de l'herpès, la métabolisation de HG1 en composés de formule I, où R est $CH_2OH$, $OCH_3$ ou COOH, ne s'effectue pas. Ceci explique que, après administration de HG1 par voie locale, les propriétés antivirales bénéfiques des composés de formule I, où R est $OCH_3$, $CH_2OH$ ou COOH, n'ont jamais pu se manifester.

On sait enfin que l'acide 3,5-di-t.-butyl-4-hydroxybenzoique, qui est un composé de formule I où R est COOH, a été utilisé comme intermédiaire de synthèse (voir en particulier l'exemple 1 de EP-A-0 269 981) et que les halogénures de cet acide, notamment les bromure et chlorure ont également été utilisés comme intermédiaires de synthèse (voir EP-A-0 269 981 précité et US-A-4 708 966).

### BUT DE L'INVENTION

On vient de trouver de façon surprenante que les composés de formule I sont particulièrement intéressants en tant qu'agents antiviraux eu égard à leurs effets inhibiteurs (i.e. virustatiques) et/ou virulicides (i.e. virucides) vis-à-vis des virus, en particulier vis-à-vis des virus à capside lipidiques (en abrégé LCV), comme les virus de l'herpès, mais aussi vis-à-vis d'autres virus et notamment des papillomavirus.

Selon un premier aspect de l'invention, on se propose de fournir une nouvelle utilisation des composés de formule I en tant qu'agents antiviraux.

Selon un second aspect de l'invention, on se propose de fournir une composition thérapeutique antivirale renfermant au moins un composé de formule I.

Selon un troisième aspect de l'invention, on se propose de fournir une nouvelle méthode de préparation desdits composés de formule I.

### OBJET DE L'INVENTION

Plus précisément on propose, selon l'invention, une nouvelle utilisation thérapeutique caractérisée en ce que l'on fait appel à un composé 2,6-di-t-butylphénol substitué en position 4 (autre nomenclature : 3,5-di-t-butyl-4-hydroxybenzène substitué en position 1) choisi parmi l'ensemble constitué par

(a) les phénols de formule

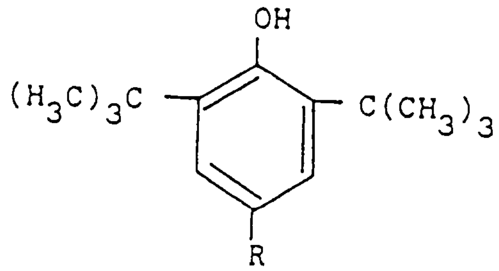

(I)

dans laquelle R est un groupe COOH ou un groupe A-COOH où A est un reste hydrocarboné aliphatique en $C_1$-$C_{11}$, et

(b) leurs sels et esters correspondants

pour l'obtention d'un médicament antiviral destiné à une utilisation en thérapeutique humaine ou vétérinaire vis-à-vis des maladies provoquées par les virus.

Cette utilisation est notamment appropriée au traitement local des maladies ou infections de la peau provoquées par lesdits virus.

Plus spécialement, l'invention propose l'utilisation anti-virale du composé phénol substitué suivant :

- l'acide 3,5-di-t-butyl-4-hydroxybenzoïque,

On préconise également selon l'invention une composition antivirale caractérisée en ce qu'elle renferme, en association avec un excipient physiologiquement acceptable, au moins un composé de formule I.

Bien entendu, une telle composition renfermera une quantité thérapeutiquement active de composant anti-viral de formule I.

Les composés de formule I, dans laquelle R est COOH et les sels et esters correspondants sont particulièrement utiles en tant que substances anti-virales vis-à-vis des infections provoquées par les LCV tels que notamment les virus de l'herpès $HSV_1$, $HSV_2$ et $HSV_1R$.

Ainsi l'invention se propose de fournir une nouvelle utilisation des composés de formule I cités ci-dessus pour l'obtention d'un médicament antiviral destiné a une utilisation en thérapeutique vis-à-vis des maladies provoquées par les virus à capside lipidique et plus particulièrement les virus de l'herpès.

Les composés de formule I, dans laquelle R est $OCH_3$, $CH_2OH$, CHO ou COOH et les sels et esters correspondants quand R est COOH peuvent être préparés selon une méthode connue en soi, par application de mécanismes réactionnels classiques. Le procédé que l'on préconise selon l'invention consiste à faire réagir un 2,4-di-t-butyl-4-halogénophénol [autre nomenclature : 3,5-di-t-butyl-4-hydroxy-halogénobenzène] de formule

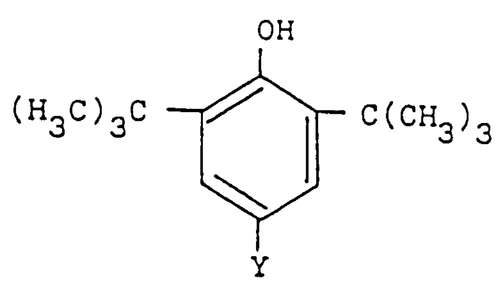

(II)

où Y est un atome d'halogène, de préférence Br ou Cl, avec un composé de formule

H-R' (III)

où R' est $OCH_3$, $CH_2OH$, CHO, COOH, COOX ou COZ,

X étant $NH_4^+$ ou $1/mM^{m+}$, M représentant un métal des groupes Ia, Ib, IIa et IIb de la classification périodique et m sa valence, et

Z étant un reste alkyle ou aminoalkyle où chaque groupe alkyle est en $C_1$-$C_5$,
en présence d'un métal alcalin, d'un hydroxyde de métal alcalin ou d'un hydrure alcalin.

Dans la mise en oeuvre de ce procédé, si cela est nécessaire le groupe OH de la fonction phénolique sera protégé selon un mode connu en soi, notamment en remplaçant ledit groupe OH phénolique par un groupe $OSi(CH_3)_3$, d'une part, et les groupes OH de $R'=CH_2OH$ ou COOH seront également protégés selon un mode connu en soi, d'autre part.

Les papillomavirus, qui sont des virus dépourvus de capside ou capsule lipidiques et qui ne font donc pas partie de l'ensemble des virus dits à capside lipidique, provoquent des infections virales qui se manifestent par des condylomes. En particulier, les papillomavirus humains (en abrégé HPV) sont responsables des condylomes ano-génitaux qui sont des maladies sexuellement transmissibles.

On sait que les condylomes plans ou acuminés dûs aux HPV sont très fréquents et que les thérapeutiques, qui ont été préconisées pour les traiter, (i) sont limitées, (ii) ne sont pas toujours efficaces ou (iii) ne sont pas suffisamment bien tolérées.

Parmi ces thérapeutiques connues, on sait que celles faisant appel à des méthodes physiques sont soit plus ou moins douloureuses (électrocoagulation, cryothérapie), soit coûteuses (laser $CO_2$), et exigent toutes en pratique une anesthésie, et que celles faisant appel à des agents chimiques anti-condylomes de référence, tels que la podophylline, la podophyllotoxine et le fluorouracil [i.e. 5-fluoro-2,4-(*1H,3H*)-pyrimidinedione], sont souvent irritantes et potentiellement toxiques.

Par ailleurs, quel que soit le traitement utilisé, ces thérapeutiques connues présentent encore l'inconvénient que le taux de récidive est toujours élevé en raison de recontamination ou d'infection virale latente. L'invention se propose de fournir une nouvelle solution technique pour le traitement des condylomes provoqués par les paillomavirus, permettant de pallier les inconvénients rappelés ci-dessus des solutions thérapeutiques antérieurement connues.

On vient en effet de trouver de façon surprenante, que les composés de formule I dans laquelle R est :

un groupe COOH ou un groupe A-COOH où A est un reste hydrocarboné aliphatique en $C_1$-$C_{11}$, et

leurs sels et esters correspondants sont particulièrement utiles en tant que substances anti-papillomavirus vis-à-vis des condylomes induits par les papillomavirus, et en particulier les condylomes du système uro-génital, notamment les condylomes ano-génitaux dûs aux HPV.

Ainsi l'invention se propose de fournir une nouvelle utilisation des composés de formule I cités ci-desssus pour l'obtention d'un médicament anti-papillomavirus destiné à une utilisation en thérapeutique vis-à-vis des condylomes provoqués par les papillomavirus.

Cette utilisation est notamment appropriée au traitement local des condylomes ano-génitaux provoquées par lesdits papillomavirus.

On préconise également selon l'invention une composition anti-papillomavirus caractérisée en ce qu'elle renferme, en association avec un excipient physiologiquement acceptable, au moins un composé de formule I ci-dessus.

Bien entendu, une telle composition renfermera une quantité thérapeutiquement active de composant anti-papillomavirus de formule I.

Plus spécifiquement, l'invention propose l'utilisation anti-papillomavirus des composés de formule I dans lesquels R est un groupe COOH.

**ABREVIATIONS**

Par commodité, les abréviations suivantes ont été utilisées dans la présente description.

| | |
|---|---|
| Ala = | alanine |
| Arg = | arginine |
| BG1 = | 3,5-di-t-butyl-4-hydroxyanisole, composé de formule I où R est $OCH_3$ [autre nomenclature : 2,6-di-t-butyl-4-méthoxyphénol] |
| BG2 = | 3,5-di-t-butyl-4-hydroxyphénylméthanol, composé de formule I où R est $CH_2OH$ [autre nomenclature : alcool 3,5-di-t-butyl-4-hydroxybenzylique ou 2,6-di-t-butyl-4-hydroxyméthyl-phénol] |
| BG3 = | 3,5-di-t-butyl-4-hydroxybenzaldéhyde, composé de formule I où R est CHO [autre nomenclature : 2,6-di-t-butyl-4-formylphénol] |
| BG4 = | acide 3,5-di-t-butyl-4-hydroxybenzoique, composé de formule I où R est COOH [autre nomenclature : 4-carboxy-2,6-di-t-butylphénol] |
| BHT = | composé 2,6-di-t-butylphénol de formule I où R est un reste hydrocarboné aliphatique [les lettres BHT proviennent historiquement de l'expression anglaise "butylated hydroxytoluene"] |
| Glu = | acide glutamique |
| $DI_{50}$ = | dose infectieuse 50 [i.e. dose induisant une infection virale chez 50 % des animaux (souris mâles adultes) recevant le virus d'essais] |
| EBV = | virus de Epstein Barr |
| Gly = | glycine |

| | |
|---|---|
| HG1 = | 2,6-di-t-butyl-paracrésol (i.e. BHT de formule I où R est méthyle) |
| HG4 = | 2,6-di-t-butyl-4-butylphénol (i.e. BHT de formule I où R est n-butyle) |
| HGt4 = | 2,4,6-tri-t-butylphénol (i.e. BHT de formule I où R est t-butyle) |
| HGt5 = | 2,6-di-t-butyl-4-(2,2-diméthylpropyl)phénol [i.e. BHT de formule I où R est $CH_2C(CH_3)_3$] |
| HG6 = | 2,6-di-t-butyl-4-hexylphénol (i.e. BHT de formule I où R est n-hexyle) |
| HGt8 = | 2,6-di-t-butyl-4-(1,1,3,3-tétraméthylbutyl)phénol [i.e. BHT de formule I où R est $C(CH_3)_2$-$CH_2$-$C(CH_3)_3$] |
| His = | histidine |
| HPV = | papillomavirus humain |
| $HSV_1$ = | virus herpes simplex de type 1 |
| $HSV_2$ = | virus herpes simplex de type 2 |
| $HSV_1R$ = | virus herpes simplex de type 1 résistant à l'acyclovir [substance antivirale de référence répondant à la formule développée de 2-amino-1,9-dihydro-9-[(2-hydroxyéthoxy)méthyl]-6H-purine-6-one] |
| 3Hyp = | 3-hydroxyproline |
| 4Hyp = | 4-hydroxyproline |
| Ile = | isoleucine |
| LCV = | virus à capside lipidique |
| Leu = | leucine |
| Lys = | lysine |
| MeGly = | méthylglycine (i.e. sarcosine) |
| Nle = | norleucine |
| Nva = | norvaline |
| Orn = | ornithine |
| TV = | titre infectieux vis-à-vis de l'échantillon du virus d'essai, ce titre est ici un multiple de $DI_{50}$/ml |
| ZV = | virus de Zoster |
| Phe = | phénylalanine |
| Pro = | proline |
| Prp = | propolis |
| $R_p$ = | rapport pondéral |
| RT = | température ambiante (15-20°C) |
| Val = | valine |

DESCRIPTION DETAILLEE DE L'INVENTION

Les composés de formule I selon l'invention comportent un groupe R qui est un reste hydrocarboné aliphatique, oxygéné, à chaîne hydrocarbonée liénaire ou ramifiée en $C_1$-$C_{12}$.

Parmi les groupes R en $C_1$-$C_{12}$ comportant une fonctionnalité oméga-carboxy qui conviennent on peut citer notamment les groupes -COOH et -A-COOH où A est un reste hydrocarboné aliphatique en $C_1$-$C_{11}$ tels que -COOH, -$CH_2COOH$, -$CH(CH_3)COOH$, -$C(CH_3)_2COOH$, -$CH_2CH_2CH_2COOH$, -$C(CH_3)_2CH_2COOH$, -CH=CH-COOH et -$C(CH_3)$=C $(CH_3)$-COOH.

Sans sortir du cadre de l'invention, le groupe aliphatique oxygéné R peut comporter (i) à son extrémité liée au reste phénol une fonctionnalité éther -O- ou carbonyle -CO-, et (ii) à son autre extrémité une fonction oméga-OH ou oméga-COOH, les deux extrémités étant reliées entre elles par une chaîne hydrocarbonée linéaire ou ramifiée telle que le nombre total d'atome de carbone de R soit au plus de 12.

Parmi les sels des composés de formule I, où R est COOH ou A-COOH, qui conviennent, on peut notamment mentionner les sels minéraux obtenus par réaction de l'acide de formule I où R = COOH ou A-COOH avec une base minérale. Ces sels minéraux sont des composés de formule I où R = COOX, X représentant $NH4^+$ ou un cation $1/mM^{m+}$, M étant un métal des groupes Ia, Ib, IIa et IIb de la classification périodique et m sa valence, notamment $Na^+$, $K^+$, $1/2Ca^{2+}$, $1/2Zn^{2+}$, $1/2Mg^{2+}$, $Cu^+$ ou $1/2Cu^{2+}$.

On peut également mentionner les sels d'addition obtenus par réaction d'un composé de formule I où R = COOH ou A-COOH avec une base organique, telle que notamment les alkylamines et dialkylamines (où chaque fragment alkyle est un radical en $C_1$-$C_8$ à chaîne hydrocarbonée linéaire ou ramifiée), les N-hydroxyalkylamines où le fragment alkyle est un radical divalent en $C_1$-$C_8$ à chaîne hydrocarbonée linéaire ou ramifiée); par exemple la 2-hydroxyéthylamine), les amines cycliques mononucléaires saturées ou insaturées [par exemple la pyridine, la 3-méthylpyridine, la pyrrolidine, la pipéridine, le 4-méthylpipéridine, la morpholine, la thiomorpholine, la pipérazine, la 4-méthylpipérazine, la 4-phénylpipérazine, la 4-(4-chlorophényl)pipérazine, la 4-(2-hydroxyéthyl) pipérazine et l'hexaméthylèneimine] et les aminoacides (par exemple Arg, His, Orn, Lys, Gly, Ala, Phe, Glu, Leu, Ile, Nle, Val, Nva, MeGly, Pro, 4Hyp ou 3Hyp, où chaque fonction acide desdits aminoacides est susceptible d'être bloquée selon une méthode connue de la synthèse peptidique).

Les esters des composés acides de formule I où R est COOH ou A-COOH peuvent être représentés par les formules COOZ ou A-COOZ, où A est défini comme ci-dessus et Z est un reste hydrocarboné susceptible d'être aminé. De façon avantageuse, Z comprendra un reste hydrocarboné aliphatique en $C_1$-$C_5$, et le groupe amino qu'il peut comporter sera $NH_2$, ou un des groupes monoalkylamino, dialkylamino, N-hydroxyalkylamino et amino cyclique tels que définis dans le cadre des sels d'addition ci-dessus.

Parmi les esters des composés acides de formule I où R est COOH ou A-COOH qui conviennent, on peut notamment mentionner les esters d'alkyle et d'aminoalkyle où chaque fragment alkyle est un reste hydrocarboné en $C_1$-$C_5$ linéaire ou ramifié.

D'une façon générale, on préfère l'acide BG4 (formule I, R = COOH) plutôt que ses sels et esters. On a en effet constaté que lesdits sels et esters agissent moins rapidement que l'acide correspondant en ce sens qu'ils retardent après administration le délitage de la forme acide active BG4.

Pour la même raison, on préfère les acides R = A-COOH aux sels et esters correspondants. Par ailleurs, on a pu constater que l'acide BG4 (composé de formule I où R = COOH) est plus efficace en tant qu'agent anti-papillomavirus que les acides de formule I où R est A-COOH.

Les composés antiviraux selon l'invention comprennent donc BG4 ainsi que ses sels et esters.

Parmi les sels de BG4 qui conviennent, on peut notamment mentionner les sels minéraux obtenus par réaction de l'acide de formule I où R = COOH avec une base minérale. Ces sels minéraux sont des composés de formule I où R = COOX, X représentant NH4$^+$ ou un cation $1/mM^{m+}$, M étant un métal des groupes Ia, Ib, IIa et IIb de la classification périodique et m sa valence, notamment $Na^+$, $K^+$, ½$Ca^{2+}$, ½$Zn^{2+}$, ½$Mg^{2+}$, $Cu^+$ ou ½$Cu^{2+}$.

On peut également mentionner les sels d'addition obtenus par réaction de BG4 avec une base organique, telle que notamment les alkylamines et dialkylamines (où chaque fragment alkyle est un radical en $C_1$-$C_8$ à chaîne hydrocarbonée linéaire ou ramifiée), les N-hydroxyalkylamines ou le fragment alkyle est un radical divalent en $C_1$-$C_8$ à chaîne hydrocarbonée linéaire ou ramifiée); par exemple la 2-hydroxyéthylamine), les amines cycliques mononucléaires saturées ou insaturées [par exemple la pyridine, la 3-méthylpyridine, la pyrrolidine, la pipéridine, le 4-méthylpipéridine, la morpholine, la thiomorpholine, la pipérazine, la 4-méthylpipérazine, la 4-phénylpipérazine, la 4-(4-chlorophényl)pipérazine, la 4-(2-hydroxyéthyl) pipérazine et l'hexaméthylèneimine] et les aminoacides (par exemple Arg, His, Orn, Lys, Gly, Ala, Phe, Glu, Leu, Ile, Nle, Val, Nva, MeGly, Pro, 4Hyp ou 3Hyp).

Parmi les esters de BG4 qui conviennent, on peut notamment mentionner les esters d'alkyle et d'aminoalkyle où chaque fragment alkyle est un reste hydrocarboné en $C_1$-$C_5$ linéaire ou ramifié.

D'une façon générale, on préfère l'acide BG4 plutôt que ses sels et esters. On a en effet constaté que lesdits sels et esters agissent moins rapidement que l'acide correspondant en ce sens qu'ils retardent après administration le délitage de la forme acide active BG4.

On a consigné, de façon non limitative, dans le tableau I ci-après un certain nombre représentatif de composés de formule I.

TABLEAU I

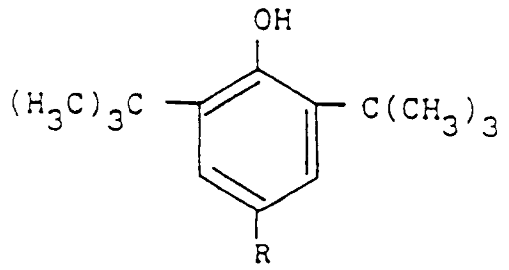

(I)

| Produit | Abréviation ou code | R |
|---|---|---|
| Ex 1 | BG1 | $OCH_3$ |
| Ex 2 | BG2 | $CH_2OH$ |
| Ex 3 | BG3 | CHO |
| Ex 4 | BG4 | COOH |
| Ex 5 | - | COONa |
| Ex 6 | - | COOH, morpholine |
| Ex 7 | - | COOH, $H_2NCH_2CH_2OH$ |
| Ex 8 | - | COOH, pyridine |
| Ex 9 | - | $COOCH(CH_3)_2$ |
| Ex 10 | - | $COOC(CH_3)_3$ |
| Ex 11 | HG1 | $CH_3$ |
| Ex 12 | HG4 | $(CH_2)_3CH_3$ |
| Ex 13 | HGt4 | $C(CH_3)_3$ |
| Ex 14 | HGt5 | $CH_2C(CH_3)_3$ |
| Ex 15 | HG6 | $(CH_2)_5CH_3$ |
| Ex 16 | HGt8 | $C(CH_3)_2CH_2C(CH_3)_3$ |
| Ex 17 | - | $CH=CH_2$ |
| Ex 18 | - | $CH=CH-CH_3$ |
| Ex 19 | - | $C(CH_3)_2CH=CHCH_3$ |
| Ex 20 | - | $C\equiv CH$ |

TABLEAU I (fin)

| Produit | Abréviation ou code | R |
|---|---|---|
| Ex 21 | - | $OCH_2CH_3$ |
| Ex 22 | - | $OC(CH_3)_3$ |
| Ex 23 | - | $OCH_2C(CH_3)_3$ |
| Ex 24 | - | $OC(CH_3)_2CH_2C(CH_3)_3$ |
| Ex 25 | - | $COCH_3$ |
| Ex 26 | - | $COCH_2CH_3$ |
| Ex 27 | - | $COCH(CH_3)_2$ |
| Ex 28 | - | $CH_2CH_2OH$ |
| Ex 29 | - | $CH_2C(CH_3)_2OH$ |
| Ex 30 | - | $C(CH_3)_2CH_2OH$ |
| Ex 31 | - | $CH_2COOH$ |
| Ex 32 | - | $C(CH_3)_2COOH$ |
| Ex 33 | - | $C(CH_3)_2CH_2COOH$ |
| Ex 34 | - | $CH(CH_3)COOH$ |
| Ex 35 | - | $CH_2COOLi$ |
| Ex 36 | - | $C(CH_3)_2COO^-$ $1/2Mg^{2+}$ |
| Ex 37 | - | $OCH_2COOH$ |
| Ex 38 | - | $OC(CH_3)_2COOH$ |
| Ex 39 | - | $COCH_2COOH$ |
| Ex 40 | - | $COCH_2CH_2OH$ |
| Ex 41 | - | $OC(CH_3)_2CH_2OH$ |
| Ex 42 | - | $OCH_2C(CH_3)_2OH$ |
| Ex 43 | - | $OCH=CH-COOH$ |

***I - Utilisation thérapeutique des composés de formule I dans laquelle R est $OCH_3$, $CH_2OH$, CHO ou COOH et les sels et esters correspondants quand R est COOH, pour l'obtention d'un médicament anti-virus de l'herpès***

Ces composés de formule I selon l'invention sont particulièrement actifs en tant qu'agents antiviraux chez l'homme et les animaux à sang chaud, notamment les mammifères, vis-à-vis des infections provoquées par les virus, en particulier les souches de virus appartenant à l'ensemble des LCV.

L'ensemble des LCV comprend notamment les EBV, $IV_A$, ZV et les virus de l'herpès.

Ces composés de formule I sont plus particulièrement intéressants vis-à-vis des souches $HSV_1$, $HSV_2$ et $HSV_1R$, dès lors que pour le traitement de l'herpès, ils peuvent facilement être conditionnés dans un excipient lipidique pour administration locale sur la peau, du type lotion, crème, onguent ou pommade, en raison de leurs propriétés oléophiles ou lipophiles.

Ces composés de formule I peuvent, comme indiqué ci-dessus, être préparés selon une méthode connue en soi, notamment par oxydation de BHT en BG2, BG3 puis BG4.

Le procédé que l'on préconise selon l'invention pour la préparation de ces composés de formule I et qui est schématisé par le mécanisme réactionnel A

$$II + III \rightarrow I$$

s'applique à la synthèse de tous ces composés de formule I. Il est avantageusement mis en oeuvre en faisant réagir dans un solvant approprié les composés II et III selon un rapport molaire II/III de préférence compris entre 1/1,2 à 1/1,9.

En variante, on peut préparer BG2 et BG4 à partir de BG3 selon un procédé schématisé par le mécanisme réactionnel B.

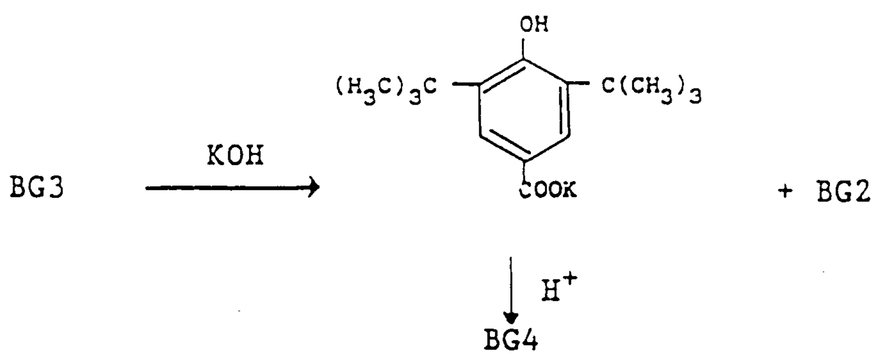


comprenant l'isolation de BG2 et BG4.

Suivant le mécanisme réactionnel B, on fait réagir BG3 avec une base forte [KOH (de préférence) ou NaOH] selon un rapport molaire BG3/base forte compris entre 1/1,3 et 1/1,8, dans un solvant approprié [notamment un solvant aromatique tel que le benzène (de préférence), le toluène ou le xylène], pendant au moins 1h à la température de reflux du mélange réactionnel. Après refroidissement jusqu'à RT, on ajoute de l'eau pour solubiliser le sel de BG4 formé. Après décantation, la phase aqueuse est extraite à l'éther. On sépare les phases aqueuse et organique, pour isoler BG2 de la phase organique et BG4 de la phase aqueuse.

En variante, on peut également préparer BG4, qui est le produit le plus intéressant de l'invention, à partir de BG3 selon un procédé schématisé par le mécanisme réactionnel C

$$BG3 \xrightarrow[H_2SO_4]{KMnO_4} BG4$$

comprenant la réaction de BG3 avec $KMnO_4$ en présence de $H_2SO_4$, pendant au moins 2 h à une température inférieure ou égale à 20°C, BG4 étant ensuite isolé du milieu réactionnel.

Le meilleur mode de mise en oeuvre selon l'invention consiste à faire appel au composé BG4 en tant que substance antivirale, notamment vis-à-vis des LCV et en particulier vis-à-vis des virus de l'herpès et plus particulièrement vis-à-vis des souches $HSV_1$, $HSV_2$ et $HSV_1R$.

D'autres avantages et caractéristiques de l'invention seront mieux compris à la lecture qui va suivre d'exemples de préparation et d'essais comparatifs.

Bien entendu, l'ensemble de ces éléments n'est nullement limitatif mais est donné à titre d'illustration.

## ESSAIS COMPARATIFS

On a apprécié les propriétés virulicides de ces composés de formule I selon l'invention par rapport au produit de comparaison A (i.e. BHT). Dans ce but, on a évalué le titre infectieux (TV) de chaque produit à tester par rapport au titre infectieux ($TV_o$) d'un échantillon de virus ("virus-stock" de souche $HSV_1$) puis calculé la valeur log ($TV/TV_o$).

Dans ce protocole, on a utilisé pour déterminer $TV_o$ une composition constituée par 0,5 ml de milieu de culture MEM et 0,5 ml de virus-stock, et pour déterminer TV une composition constituée par 0,5 ml de produit à tester dans ledit milieu MEM et 0,5 ml de virus-stock. On incube à 37°C pendant 1 h, centrifuge puis mesure les $TV_o$ et TV sur plaque de microtitration.

Les résultats obtenus sont consignés dans le tableau II ci-après où les valeurs $TV_o$ et TV sont des multiples de la $DI_{50}$/ml.

TABLEAU II

| ACTIVITE VIRULICIDE | | |
|---|---|---|
| Produit | titre infectieux (x $DI_{50}$/ml) | log($TV/TV_o$) |
| Virus | $TV_o = 10^5$ | - |
| Ex 1 (BG1) | $TV = 10^{1,5}$ | -3,5 |
| Ex 2 (BG2) | $TV = 10^{1,5}$ | -3,5 |
| Ex 3 (BG3) | $TV = 10^3$ | -2 |
| Ex 4 (BG4) | TV = 0 (a) | -5 (b) |
| A (BHT) | $TV = 10^{3,5}$ | -1,5 |

Notes
(a) éradication
(b) la valeur log($TV/TV_o$) est notée comme étant égale à -5 eu égard à l'éradication précitée.

Les résultats du tableau II mettent en évidence que la diminution du titre infectieux est la plus importante pour BG4 par rapport aux autres produits. L'activité des produits testés vis-à-vis de la souche $HSV_1$ est la suivante :

$$BG4 > BG1 = BG2 > BG3 > BHT.$$

## ESSAIS COMPLEMENTAIRES

Des essais complémentaires ont été réalisés avec BG4, le produit le plus efficace en tant qu'agent antiviral selon l'invention. Ces essais complémentaires ont été entrepris à RT (i) avec une concentration en BG4 de 0,1 % p/v pendant 1 h, et (ii) avec une concentration en BG4 de 0,5 % p/v pendant 0,5 h.

Dans le premier cas, on constate qu'il y a éradication [i.e. destruction (ou inhibition) totale des virus] en 1 h à RT, et dans le second cas, qu'il y a éradication en 0,5 h à RT.

Les résultats d'autres essais entrepris avec des doses croissantes de BG4 suivant une incubation avec l'échantillon de virus, pendant 1h à 37°C, d'une part, et pendant 0,5 h à 37°C, d'autre part, ont été consignés dans le tableau III ci-après qui confirme ladite éradication.

TABLEAU III

| Dose de BG4 (% p/v) | Incubation 1h à 37°C (*) (**) | | Incubation 0,5 h à 37°C (*) (**) | |
|---|---|---|---|---|
| virus | TVo = $10^5$ | - | TVo = $10^5$ | - |
| 5,0 | TV = 0(a) | - 5(b) | TV = 0 | - 5(b) |
| 2,5 | TV = 0(a) | - 5(b) | TV = 0 | - 5(b) |
| 1,0 | TV = 0(a) | - 5(b) | TV = 0 | - 5(b) |
| 0,50 | TV = 0(a) | - 5(b) | TV = 0 | - 5(b) |
| 0,25 | TV = 0(a) | - 5(b) | TV = $10^1$ | - 4 |
| 0,10 | TV = 0(a) | - 5(b) | TV = $10^2$ | - 3 |
| 0,050 | TV = $10^1$ | - 4 | TV = $10^3$ | - 2 |
| 0,025 | TV = $10^2$ | - 3 | TV = $10^4$ | - 1 |
| 0,010 | TV = $10^3$ | - 2 | TV = $10^4$ | - 1 |

Notes
(*) titre infectieux (x $DI_{50}$/ml)
(**) log (TV/TVo)
(a) et (b) comme dans tableau II

PREPARATION I - Formulation -

On porte à 70-75°C un mélange de 59 g de vaseline blanche, de 3 g de sesquioléate de sorbitan et de 3 g de monooléate de glycérol. On cesse de chauffer quand le mélange est devenu homogène et ajoute ensuite sous agitation 5 g de BG4. On ajoute alors 30 g d'eau à une température inférieur ou égale à 65°C et poursuit l'agitation jusqu'à refroidissement à RT. On homogénéise et obtient une pommade constituée par une émulsion eau-dans-huile, utilisable comme collyre.

**PREPARATION II - Formulation -**

On mélange à 70-75°C 57 g de vaseline blanche avec 3,6 g de sesquioléate de sorbitan, 3,6 g de monooléate de glycérol. Quand le mélange est devenu homogène, on cesse le chauffage et ajoute 5,8 g de HG1. On mélange à nouveau et ajoute sous agitation 30 g d'eau à une température inférieure à 70°C environ. On poursuit l'agitation jusqu'à refroidissement à RT, puis on homogénéise et obtient une pommade constituée par une émulsion eau-dans-huile, utilisable comme collyre.

**PREPARATION III** - Formulation -

On mélange 4,7 g de stéarate de polyéthylèneglycol 1500 avec 13 g de monostéarate de glycérol, 3 g de monooléate de glycérol, 10,5 g d'oléate de décyle, 5,5 g de triglycéride caprique/caprylique et 5 g d'isostéarate de glycérol. On chauffe progressivement jusqu'à 70-75°C et on cesse le chauffage dès que le mélange est devenu homogène. On ajoute alors 8 g de BG4 et agite lentement. On verse alors dans le mélange résultant 3 g de propylèneglycol, 0,3 g de citral et 47 g d'eau. On agite le mélange résultant tout en le laissant se refroidir jusqu'à RT. Après passage dans un dispositif d'homogénéisation, on obtient une émulsion eau-dans-huile ayant la consistance d'une crème.

**PREPARATION IV**

- Obtention de BG4 selon le mécanisme réactionnel B -

a) Dans un ballon équipé d'une ampoule d'introduction de réactif liquide ("ampoule à brome") et d'un réfrigérant on charge une solution de 10 g (43 mmoles) de 3,5-di-t-butyl-4-hydroxybenzaldéhyde (BG3) dans 20 ml de benzène. On ajoute par l'ampoule à brome 6,6 ml d'une solution aqueuse de KOH à 60 % p/v [soit 3,96 g (71 mmoles) de KOH] et agite énergiquement jusqu'à l'obtention d'une émulsion homogène. Le mélange réactionnel résultant est porté au reflux pendant une nuit (soit environ 8-14 h). Après refroidissement, on ajoute 15 ml d'eau pour solubiliser le sel de potassium de l'acide 3,5-di-t-butyl-4-hydroxybenzoïque qui s'est formé. Après décantation, on recueille la

phase aqueuse que l'on extrait au diéthyléther (3 x 15 ml). On traite ensuite séparément la phase aqueuse, d'une part, et les phases organiques réunies (lesdites phases organiques réunies seront utilisées dans la préparation V ci-après), d'autre part.

b) On agite la phase aqueuse avec 5 ml de bisulfite de sodium, puis acidifie avec HCl dilué au tiers. Le précipité qui se forme est filtré, lavé jusqu'à neutralité, séché à l'étuve à 75°C puis purifié sur colonne de gel de silice en utilisant en tant qu'éluant un mélange diéthyléther/éther de pétrole 30/70 v/v. On obtient 3,33 g (rendement : 62,4 %) du produit attendu, l'acide 3,5-di-t-butyl-4-hydroxybenzoïque. F = 205-208°C.

**PREPARATION V**

- Obtention de BG2 selon le mécanisme réactionnel B -

Les phases organiques réunies, obtenues selon la préparation IVa, sont agitées avec 5 ml de bisulfite de sodium. On lave ensuite successivement avec de l'eau (20 ml), du bicarbonate de sodium (5 ml) puis à nouveau de l'eau (2 x 20 ml). Après séchage sur $MgSO_4$, les solvants sont évaporés sous vide et le résidu d'évaporation est purifié sur une colonne de gel de silice en utilisant en tant qu'éluant un mélange diéthyléther/éther de pétrole 20/80 v/v. On obtient 3,29 g (rendement : 65,3 %) du produit attendu, le 3,5-di-t-butyl-4-hydroxyphénylméthanol.

**PREPARATION VI**

- Obtention de BG4 selon le mécanisme réactionnel C -

Dans un ballon équipé d'une ampoule d'introduction de réactif liquide ("ampoule à brome") et d'un réfrigérant, et placé dans un bain d'eau-glace, on prépare une solution aqueuse d'acide $H_2SO_4$ en utilisant 40 ml d'eau et 6,5 g d'$H_2SO_4$ concentré. Quand la température de ladite solution descend jusqu'à 15°C, on ajoute sous agitation 7,5 g (32 mmoles) de 3,5-di-t-butyl-4-hydroxybenzaldéhyde puis ensuite 3,6 g (23 mmoles) de $KMnO_4$ par petites portions. L'addition de $KMnO_4$ est effectuée de telle façon que, au cours de ladite addition, la température du milieu réactionnel ne dépasse pas 20°C. L'addition de $KMnO_4$ étant terminée, on poursuit l'agitation pendant 6h à une température inférieure à 20°C.

On ajoute ensuite goutte à goutte une solution aqueuse de bisulfite de sodium pour dissoudre le $MnO_2$ formé (disparition de la coloration brun foncé). Le précipité restant est filtré, lavé avec de l'eau jusqu'à neutralité et solubilisé dans 20 ml d'une solution aqueuse de NaOH à 20 % p/v. La solution aqueuse constituant le filtrat est lavée à l'éther, puis acidifiée avec HCl dilué (au tiers). Il se forme un nouveau précipité qui est recueilli, lavé avec de l'eau jusqu'à neutralité, séché à l'étuve à 75°C puis purifié sur une colonne de gel de silice en utilisant en tant qu'éluant un mélange diéthyléther/éther de pétrole 30/70 v/v. On obtient 4,67 g (rendement : 58,3 %) du produit attendu, l'acide 3,5-di-t-butyl-4-hydroxybenzoïque. F = 205-208°C.

**PREPARATION VII**

- Obtention de BG1 selon le mécanisme réactionnel A -

On fait réagir 10 mmoles de 2,4-di-t-butyl-4-bromophénol [autre nomenclature : 3,5-di-t-butyl-4-hydroxy-bromobenzène] avec 17 mmoles de méthanol, dans un solvant approprié (notamment le tétrahydrofuranne) en présence de NaOH. On obtient (rendement : 62 %) le produit attendu, le 3,5-di-t-butyl-4-hydroxyanisole.

**PREPARATION VIII**

- Obtention de BG3 selon le mécanisme réactionnel A -

En procédant comme indiqué dans la préparation VII ci-dessus, à partir de 10 mmoles de 3,5-di-t-butyl-4-triméthylsilyloxy-bromobenzène et de 15 mmoles de HCHO [sous la forme protégée de $H_2C(OCH_3)_2$] on obtient (rendement : 45 %) le 3,5-di-t-butyl-4-triméthylsilyloxybenzaldéhyde puis le produit attendu, le 3,5-di-t-butyl-4-hydroxybenzaldéhyde.

***II - Utilisation thérapeutique des composés de formule I dans laquelle R est un groupe alcyle en $C_1$-$C_{12}$, un groupe alcényle en $C_2$-$C_{12}$, un groupe alcynyle en $C_2$-$C_{12}$, un groupe alkoxy en $C_1$-$C_{12}$, un groupe formyle, un groupe alkanoyle en $C_2$-$C_{12}$, un groupe hydroxyalkyle en $C_1$-$C_{12}$, un groupe COOH ou un groupe A-COOH où A est un reste hydrocarboné aliphatique en $C_1$-$C_{11}$ et leurs sels et esters correspondants quand R est COH ou A-COOH pour l'obtention d'un médicament anti-papillomavirus.***

Ces composés de formule I sont actifs vis-à-vis des papillomavirus et utiles en tant qu'agents anti-papillomavirus dans le traitement des condylomes dûs aux HPV.

Leur activité anti-papillomavirus est améliorée quand ils sont associés à la propolis (Prp). On a en effet découvert que la synergie de l'association BHT/Prp, déjà mise en évidence vis-à-vis des LCV, tels que $HSV_1$, $HSV_2$ et $HSV_1R$, avec un rapport pondéral BHT/Prp compris entre 100/1 et 650/1 selon la demande de brevet français No 90 03 093 précitée, s'applique également à l'association composé de formule I/Prp vis-à-vis des papillomavirus et notamment de HPV, et en particulier dans le traitement des condylomes dûs aux HPV.

En bref, dans l'organisme, les composés de formule I et leurs associations synergétiques avec Prp, interviennent en tant que moyens virustatiques ou virucides selon la dose utilisée, et manifestent des propriétés anti-tumorales.

**MEILLEUR MODE**

Selon le meilleur mode de mise en oeuvre de l'invention, pour le traitement des lésions de muqueuses ano-génitales que constituent les condylomes acuminés ou plans provoqués par les HPV, on fera appel à l'un des composés suivants : BG1, BG2, BG4, HG1, HG4, HGt4, HG6 ou HGt8, le cas échéant en association avec Prp.

Les composés préférés selon l'invention sont BG4 et HG1.

**POSOLOGIE**

La posologie, que l'on préconise pour le traitement des condylomes ano-génitaux provoqués par les HPV, consiste à administrer au niveau de lésions des muqueuses ano-génitales une composition locale (i.e. dermatologique) renfermant 5 à 25 % en poids d'au moins un composé de formule I.

De façon pratique, on utilisera une composition locale renfermant

(a) 20 % en poids de HG1,
(b) de la propolis et 10 % en poids de HG1, avec un $R_p$ HG1/Prp compris entre 135/1 et 560/1,
(c) 10 % en poids de BG4, ou
(d) de la propolis et 5-7 % de BG4, avec un $R_p$ BG4/Prp compris entre 135/1 et 560/1.

D'autres avantages et caractéristiques de l'invention seront mieux compris à la lecture qui va suivre d'exemples de préparation et d'essais comparatifs.

Bien entendu, l'ensemble de ces éléments n'est nullement limitatif mais est donné à titre d'illustration.

I. - **ESSAIS COMPARATIFS**

On a résumé ci-après les essais comparatifs cliniques qui ont été entrepris en double insu (i.e. double aveugle) sur des patients des deux sexes agés de 18 ans ou plus et répartis en cinq lots :

- <u>lot témoin</u> recevant uniquement une préparation dermatologique constituée d'excipients sous forme d'onguent;
- <u>lot A</u> recevant la préparation dermatologique du lot témoin dans laquelle avait été incorporé le produit HG1 à la concentration de 20 % en poids;
- <u>lot B</u> recevant la préparation dermatologique du lot témoin dans laquelle avait été incorporé le produit HG1 à la concentration de 10 % en poids;
- <u>lot C</u> recevant la préparation dermatologique du lot témoin dans laquelle avait été incorporé un mélange HG1/Prp (selon un $R_p$ de 270/1), la concentration de HG1 dans cette préparation dermatologique étant de 10 % en poids; et
- <u>lot D</u> recevant la préparation dermatologique du lot témoin dans laquelle avait été incorporé le produit BG4 à la concentration de 10 % en poids.

Les patients, tous volontaires, présentaient tous des condylomes ano-génitaux végétants ou papuleux et n'avaient reçu aucun traitement anti-HPV au moins pendant les 15 jours précédents le début de la présente expérimentation. La majeure partie des patients était des hommes présentant principalement des lésions localisées sur la verge (notamment le fourreau). Il y avait autant de patients avec des lésions papuleuses que de patients avec des lésions végétantes. Approximativement, un tiers des patients présentait 1 ou 2 condylomes (i.e. lésions), un tiers 3 à 5 condylomes et un

tiers au moins 6 condylomes. Dans deux cas sur trois, il s'agissait d'une récidive; les patients présentant une récidive avaient été traités auparavant par azote liquide, laser $CO_2$, podophylline ou podophyllotoxine.

Chaque patient des lots témoins, A, B, C et D a reçu deux applications quotidiennes locales au niveau des lésions pendant 14 jours. On a noté avant et après traitement le nombre de lésions pour apprécier l'efficacité du traitement.

Les caractères démographiques et cliniques des patients des lots témoin, A, B, C et D avant traitement figurent dans le tableau IV ci-après. L'évolution du nombre de lésions par lot a été consignée dans le tableau V ci-après.

TABLEAU IV

| CARACTERES DEMOGRAPHIQUES ET CLINIQUES DES PATIENTS AVANT TRAITEMENT | | | | | |
|---|---|---|---|---|---|
| | lot témoin | lot A | lot B | lot C | lot D |
| nombre de patients | 14 | 14 | 13 | 15 | 14 |
| - hommes | 11 | 12 | 13 | 12 | 11 |
| - femmes | 3 | 2 | 0 | 3 | 3 |
| âge moyen (ans) | 27,9 | 27,7 | 29,3 | 28,2 | 29,4 |
| localisation des lésions (1) | | | | | |
| verge | 10 | 12 | 13 | 12 | 12 |
| vulve | 2 | 2 | 0 | 3 | 3 |
| anus | 3 | 0 | 1 | 2 | 1 |
| type de condylome acuminé/plan | 7/7 | 6/8 | 8/5 | 8/7 | 7/7 |
| nombre de lésions | | | | | |
| 1 ou 2 | 4 | 5 | 4 | 6 | 4 |
| 3 à 5 | 3 | 4 | 6 | 6 | 4 |
| 6 ou plus | 7 | 4 | 3 | 3 | 6 |

TABLEAU V

| EVOLUTION DU NOMBRE DE LESIONS APRES 14 JOURS DE TRAITEMENT | | | | | |
|---|---|---|---|---|---|
| Nombre de lésion | lot témoin | lot A | lot B | lot C | lot D |
| a) échec dont | 12 | 2 | 6 | 2 | 1 |
| - augmentation | 4 | 0 | 0 | 0 | 0 |
| - stable | 8 | 2 | 6 | 2 | 1 |
| b) succès dont | 2 | 12 | 7 | 13 | 13 |
| - diminution | 2 | 8 | 6 | 7 | 6 |
| - guérison | 0 | 4 | 1 | 6 | 7 |

On a constaté que les produits selon l'invention utilisés dans les lots A, B, C et D étaient bien tolérés (on n'a observé que deux cas d'irritation uniquement dans le lot A recevant la composition locale renfermant 20 % en poids de HG1) et efficaces de façon statistiquement significative par rapport au lot témoin. Parmi les lots A, B et C recevant HG1 (à 20 %, 10 % et respectivement 10 % en association avec Prp), on a constaté que HG1 à la dose de 20 % (lot A) est plus efficace qu'à la dose de 10 % (lot B) et que l'association HG1/Prp où le HG1 est à la dose de 10 % (lot C) est au moins, voire plus, efficace que HG1 à la dose de 20 % (lot A), d'une part, et mieux toléré (absence d'irritation) que HG1 à la dose de 20 % (lot A), d'autre part.

On a également constaté que BG4 à la dose de 10 % en poids (lot D) est au moins aussi efficace que l'association HG1/Prp (lot C).

II. - **ESSAIS COMPLEMENTAIRES**

Les patients du lot témoin des essais comparatifs précédents ont été traités comme indiqué ci-dessus avec une composition comprenant la préparation dermatologique d'excipients des essais I ci-dessus contenant une association BG4/Prp avec un $R_p$ de 270/1, BG4 étant à une concentration de 5 % en poids.

Les résultats obtenus sont similaires ou supérieurs à ceux du lot D des essais I ci-dessus.

III. - **FORMULATION AVEC HG1**

On mélange 4 g de stéarate de polyéthylèneglycol 1500 avec 12 g de monostéarate de glycérol, 3 g de monooléate de glycérol, 10 g d'oléate de décyle, 5 g de triglycéride caprique/caprylique et 5 g d'isostéarate de glycérol. On chauffe progressivement jusqu'à 70-75°C et on cesse le chauffage dès que le mélange est devenu homogène. On ajoute alors 20 g de HG1 et agite lentement. On verse alors dans le mélange résultant 3 g de propylèneglycol et 38 g d'eau. On agite le mélange résultant tout en le laissant se refroidir jusqu'à RT. Après passage dans un dispositif d'homogénéisation, on obtient une émulsion eau-dans-huile ayant la consistance d'une crème.

IV. - **FORMULATION AVEC HG1**

On mélange 4 g de stéarate de polyéthylèneglycol 1500 avec 12 g de monostéarate de glycérol, 3 g de monooléate de glycérol, 10,3 g d'oléate de décyle, 5 g de triglycéride caprique/caprylique et 5 g d'isostéarate de glycérol. On chauffe progressivement jusqu'à 70-75°C et on cesse le chauffage dès que le mélange est devenu homogène. On ajoute alors 10 g de HG1 et agite lentement. On verse alors dans le mélange résultant 3 g de propylèneglycol et 47 g d'eau. On agite le mélange résultant tout en le laissant se refroidir jusqu'à RT. Après passage dans un dispositif d'homogénéisation, on obtient une émulsion eau-dans-huile ayant la consistance d'une crème.

V. - **FORMULATION AVEC BG4**

On mélange 4,7 g de stéarate de polyéthylèneglycol 1500 avec 12 g de monostéarate de glycérol, 3 g de monooléate de glycérol, 10,3 g d'oléate de décyle, 5 g de triglycéride caprique/caprylique et 5 g d'isostéarate de glycérol. On chauffe progressivement jusqu'à 70-75°C et on cesse le chauffage dès que le mélange est devenu homogène. On ajoute alors 10 g de BG4 et agite lentement. On verse alors dans le mélange résultant 3 g de propylèneglycol et 47 g d'eau. On agite le mélange résultant tout en le laissant se refroidir jusqu'à RT. Après passage dans un dispositif d'homogénéisation, on obtient une émulsion eau-dans-huile ayant la consistance d'une crème.

VI. - **FORMULATION AVEC HGt4 et Prp**

On mélange 4,66 g de stéarate de polyéthylèneglycol 1500 avec 12 g de monostéarate de glycérol, 3 g de monooléate de glycérol, 10 g d'oléate de décyle, 5 g de triglycéride caprique/caprylique et 5 g d'isostéarate de glycérol. On chauffe progressivement jusqu'à 70-75°C et on cesse le chauffage dès que le mélange est devenu. homogène. On ajoute alors 10 g de HGt4 et agite lentement. On verse alors dans le mélange résultant 3 g de propylèneglycol, 0,3 g de citral, 0,04 g de Prp ($R_p$ : 250/1) et 47 g d'eau. On agite le mélange résultant tout en le laissant se refroidir jusqu'à RT. Après passage dans un dispositif d'homogénéisation, on obtient une émulsion eau-dans-huile ayant la consistance d'une crème.

VII. - **FORMULATION AVEC HG1 et Prp**

On mélange 4,66 g de stéarate de polyéthylèneglycol 1500 avec 12 g de monostéarate de glycérol, 3 g de monooléate de glycérol, 10 g d'oléate de décyle, 5,8 g de triglycéride caprique/caprylique et 5 g d'isostéarate de glycérol. On chauffe progressivement jusqu'à 70-75°C et on cesse le chauffage dès que le mélange est devenu homogène. On ajoute alors 10 g de HG1 et agite lentement. On verse alors dans le mélange résultant 3 g de propylèneglycol, 0,04 g de Prp ($R_p$ = 250/1) et 47 g d'eau. On agite le mélange résultant tout en le laissant se refroidir jusqu'à RT. Après passage dans un dispositif d'homogénéisation, on obtient une émulsion eau-dans-huile ayant la consistance d'une crème.

**Revendications**

1. Utilisation d'un composé phénol caractérisée en ce que l'on fait appel à un composé 2,6-di-t-butylphénol substitué en position 4 choisi parmi l'ensemble constitué par les phénols de formule :

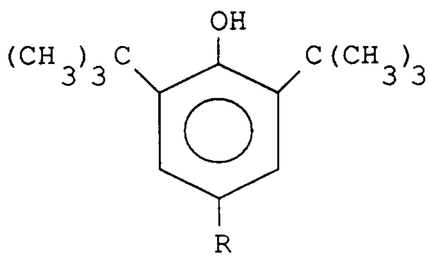

(I)

dans laquelle R est un groupe COOH , un groupe A-COOH, où A est un reste hydrocarboné aliphatique en $C_1$-$C_{11}$ , et leurs sels et esters correspondants ,

pour l'obtention d'un médicament antiviral destiné à une utilisation en thérapeutique humaine ou vétérinaire vis-à-vis des maladies provoquées par les virus.

2. Utilisation thérapeutique d'un composé 2,6-di-t-butylphénol substitué en position 4 selon la revendication 1, caractérisée en ce qu'elle est destinée à l'obtention d'un médicament utile vis-à-vis des maladies provoquées par les virus à capside lipidique.

3. Utilisation thérapeutique d'un composé 2,6-di-t-butylphénol substitué en position 4 selon la revendication 2, caractérisée en ce qu'elle est destinée à l'obtention d'un médicament utile vis-à-vis des maladies provoquées par les virus de l'herpès.

4. Utilisation suivant l'une quelconque des revendications 2 et 3, caractérisée en ce que ledit composé 2,6-di-t-butylphénol substitué en position 4 intervenant en tant qu'agent anti-viral est administré par voie locale dans le cas d'une infection de la peau provoquée par lesdits virus.

5. Utilisation d'un composé 2,6-di-t-butylphénol substitué en position 4 selon la revendication 1, caractérisée en ce qu'elle est destinée à l'obtention d'un médicament utile vis-à-vis des maladies provoquées par les papillomavirus.

6. Utilisation thérapeutique d'un composé 2,6-di-t-butylphénol substitué en position 4 selon la revendication 5, caractérisée en ce qu'elle est destinée à l'obtention d'un médicament utile vis-à-vis des condylomes provoqués par les papillomavirus.

7. Utilisation thérapeutique d'un composé 2,6-di-t-butylphénol substitué en position 4 selon la revendication 5, caractérisée en ce qu'elle est detinée à l'obtention d'un médicament utile vis-à-vis des condylomes ano-géntitaux dûes aux papillomavirus humains.

8. Utilisation selon l'une quelconque des revendications 5 à 7, caractérisée en ce que la propolis est associé au composé 2,6-di-t-butylphénol substitué en position 4.

9. Utilisation selon l'une quelconque des revendications 1 à 8, caractérisée en ce que ledit composé 2,6-di-t-butylphénol substitué en position 4 est l'acide 2,6-di-t-butyl-4-hydroxybenzoïque.

10. Composition antivirale, caractérisée en ce qu'elle renferme, en association avec un excipient physiologiquement acceptable, au moins un composé de formule I, selon la revendication 1.

11. Composition antivirale utile vis-à-vis des maladies provoquées par les virus de l'herpès, caractérisée en ce qu'elle renferme, en association avec un excipient physiologiquement acceptable, au moins un composé de formule I.

12. Composition thérapeutique pour application locale sur la peau dans le cas d'une infection de celle-ci provoquée par les virus de l'herpès, caractérisée en ce qu'elle renferme, en association avec un excipient physiologiquement acceptable, au moins un composé de formule I.

13. Composition thérapeutique pour application locale sur les lésions constituées par les condylomes ano-génitaux, caractérisée en ce qu'elle renferme, en association avec un excipient physiologiquement acceptable, au moins un composé de formule I.

14. Composition thérapeutique selon la revendication 13, caractérisée en ce qu'elle renferme en outre de la propolis selon un rapport pondéral, composé de formule I/propolis compris entre 100/1 et 50/1.

## Claims

1. Use of a phenol compound, characterized in that a 4-position substituted 2,6-di-t-butylphenol is employed which is selected among the group constituted by phenols of the formula :

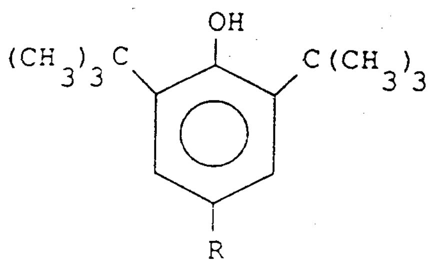

(I)

wherein R is a group COOH, a group A-COOH wherein A is an aliphatic $C_1$-$C_{11}$ hydrocarbon residue, and corresponding salts and esters thereof, for obtaining an antivirus drug adapted to be used in human or veterinary therapy against virus-induced diseases.

2. The therapeutic use of a 4-position substituted 2,6-di-t-butylphenol compound as recited in claim 1, characterized in that it is adapted to obtaining a useful drug against lipidic capside virus induced diseases.

3. The therapeutic use of a 4-position substituted 2,6-di-t-butylphenol compound as recited in claim 2, charactérized in that it is adapted to obtaining a useful drug against herpes virus induced diseases.

4. The use as recited in any claim 2 and 3, characterized in that said 4-position substituted 2,6-di-t-butylphenol compound acting as an anti-virus agent is topically administered should a skin infection occur due to said virus.

5. The use of a 4-position substituted 2,6-di-t-butylphenol compound as recited in claim 1, characterized in that it is adapted to obtaining a useful drug against papillomavirus induced diseases.

6. The therapeutic use of a 4-position substituted 2,6-di-t-butylphenol compound as recited in claim 5, characterized in that it is adapted to obtaining a useful drug against papillomavirus induced condylomata.

7. The therapeutic use of a 4-position substituted 2,6-di-t-butylphenol compound as recited in claim 5, characterized in that it is designed to obtaining a useful drug against human papillomavirus-induced ano-genital condylomata.

8. The use as recited in any claim 5-7, characterized in that propolis is associated with the 4-position substituted 2,6-di-t-butylphenol compound.

9. The use as recited in any claim 1-8, characterized in that said 4-position substituted 2,6-di-t-butylphenol compound is the 2,6-di-t-butyl-4-hydroxybenzoic acid.

10. An antivirus composition, characterized in that it includes, in association with a physiologically acceptable excipient, at least a compound of formula I, according to claim 1.

11. A useful antivirus composition against herpes virus induced diseases, characterized in that it includes, in association with a physiologically acceptable excipient, at least a compound of formula I.

12. A therapeutic composition to be topically applied upon skin in case of an infection thereof due to herpes virus, characterized in that it includes, in association with a physiologically acceptable excipient, at least a compound of formula I.

13. A therapeutic composition to be topically applied upon lesions due to ano-genital condylomata, characterized in that it includes, in association with a physiologically acceptable excipient, at least a compound of formula I.

14. The therapeutic composition of claim 13, characterized in that it further includes propolis according to a weight ration between formula I compound and propolis in the range from 100/1 to 50/1.

**Patentansprüche**

1. Verwendung einer Phenolverbindung, dadurch gekennzeichnet, daß eine in 4-Stellung substituierte 2,6-Di-t-butylphenolverbindung eingesetzt wird, ausgewählt aus der Gruppe, bestehend aus den Phenolen der Formel:

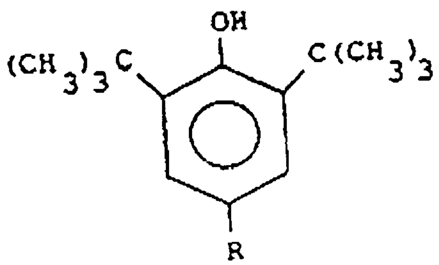

(I)

,

worin R für eine COOH-Gruppe, eine A-COOH-Gruppe,

worin A für einen aliphatischen $C_1$-$C_{11}$-Kohlenwasserstoffrest steht, und für ihre entsprechenden Salze und Ester steht,

zur Herstellung eines antiviralen Medikaments, das zur Verwendung in der Human- oder Veterinärtherapie gegen durch Viren hervorgerufene Krankheiten bestimmt ist.

2. Therapeutische Verwendung einer in 4-Stellung substituierten 2,6-Di-t-butylphenolverbindung nach Anspruch 1, dadurch gekennzeichnet, daß sie zur Herstellung eines antiviralen Medikaments bestimmt ist, das gegen durch Viren mit einem Lipid-Capsid hervorgerufene Krankheiten geeignet ist.

3. Therapeutische Verwendung einer in 4-Stellung substituierten 2,6-Di-t-butylphenolverbindung nach Anspruch 2, dadurch gekennzeichnet, daß sie zur Herstellung eines Medikaments bestimmt ist, das gegen durch Herpes-Viren hervorgerufene Krankheiten geeignet ist.

4. Verwendung nach einem der Ansprüche 2 und 3, dadurch gekennzeichnet, daß die genannte, in 4-Stellung substituierte 2,6-Di-t-butylphenolverbindung, die als antivirales Mittel wirkt, im Falle einer durch die genannten Viren hervorgerufene Infektion der Haut lokal verabreicht wird.

5. Verwendung einer in 4-Stellung substituierten 2,6-Di-t-butylphenolverbindung nach Anspruch 1, dadurch gekennzeichnet, daß sie zur Herstellung eines Medikaments bestimmt ist, das gegen durch Papillomaviren hervorgerufene Krankheiten geeignet ist.

6. Therapeutische Verwendung einer in 4-Stellung substituierten 2,6-Di-t-butylphenolverbindung nach Anspruch 5, dadurch gekennzeichnet, daß sie zur Herstellung eines Medikaments bestimmt ist, das gegen durch Papillomaviren hervorgerufene Kondylome geeignet ist.

7. Therapeutische Verwendung einer in 4-Stellung substituierten 2,6-Di-t-butylphenolverbindung nach Anspruch 5, dadurch gekennzeichnet, daß sie zur Herstellung eines Medikaments bestimmt ist, das gegen Anogenital-Kondylome aufgrund menschlicher Papillomaviren geeignet ist.

8. Verwendung nach einem der Ansprüche 5 bis 7, dadurch gekennzeichnet, daß Propolis mit der in 4-Stellung substituierten 2,6-Di-t-butylphenolverbindung assoziiert ist.

9. Verwendung nach einem der Ansprüche 1 bis 8, dadurch gekennzeichnet, daß die genannte, in 4-Stellung substituierte 2,6-Di-t-butylphenolverbindung 2,6-Di-t-butyl-4-hydroxybenzoesäure ist.

10. Antivirale Zusammensetzung, dadurch gekennzeichnet, daß sie in Verbindung mit einem physiologisch verträglichen Hilfsstoff wenigstens eine Verbindung der Formel I nach Anspruch 1 einschließt.

11. Antivirale Zusammensetzung, die gegen durch Herpes-Viren hervorgerufene Krankheiten geeignet ist, dadurch gekennzeichnet, daß sie in Verbindung mit einem physiologisch verträglichen Hilfsstoff wenigstens eine Verbindung der Formel I einschließt.

12. Therapeutische Zusammensetzung zur lokalen Verabreichung auf der Haut im Falle einer Infektion von ihr, die durch Herpes-Viren hervorgerufene wird, dadurch gekennzeichnet, daß sie in Verbindung mit einem physiologisch verträglichen Hilfsstoff wenigstens eine Verbindung der Formel I einschließt.

13. Therapeutische Zusammensetzung zur lokalen Verabreichung auf Läsionen, bestehend aus Anogenital-Kondylomen, dadurch gekennzeichnet, daß sie in Verbindung mit einem physiologisch verträglichen Hilfsstoff wenigstens eine Verbindung der Formel I umfaßt.

14. Therapeutische Zusammensetzung nach Anspruch 13, dadurch gekennzeichnet, daß sie ferner Propolis in einem Gewichtsverhältnis von Verbindung der Formel I/Propolis zwischen 100/1 und 50/1 umfaßt.